(19) **Europäisches Patentamt / European Patent Office / Office européen des brevets**

(11) **EP 1 825 857 A1**

(12) **EUROPEAN PATENT APPLICATION**
published in accordance with Art. 158(3) EPC

(43) Date of publication:
**29.08.2007 Bulletin 2007/35**

(51) Int Cl.:
*A61K 31/7016* (2006.01)    *A61K 31/702* (2006.01)
*A61K 31/7032* (2006.01)    *A61K 31/715* (2006.01)
*A61K 31/047* (2006.01)    *A61K 33/06* (2006.01)
*A61P 1/10* (2006.01)    *A61P 35/00* (2006.01)
*A23L 1/30* (2006.01)    *A23L 1/304* (2006.01)

(21) Application number: **05806886.7**

(22) Date of filing: **18.11.2005**

(86) International application number:
**PCT/JP2005/021272**

(87) International publication number:
**WO 2006/054710 (26.05.2006 Gazette 2006/21)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **22.11.2004 JP 2004368458**
**06.05.2005 JP 2005134745**

(71) Applicant: **SUNSTAR INC.**
**Takatsuki-shi,**
**Osaka 569-1195 (JP)**

(72) Inventors:
• **SATO, Takehiko,**
**c/o SUNSTAR INC.**
**Takatsuki-shi, Osaka 5691195 (JP)**
• **ISHIKADO, Atsushi,**
**c/o SUNSTAR INC.**
**Takatsuki-shi, Osaka 5691195 (JP)**

(74) Representative: **TBK-Patent**
**Bavariaring 4-6**
**80336 München (DE)**

(54) **AGENT FOR AMELIORATING THE MAXIMUM PASSAGE TIME THROUGH DIGESTIVE TRACT, AGENT FOR AMELIORATING PASSAGE TIME THROUGH DIGESTIVE TRACT AND PREVENTIVE FOR COLON CANCER**

(57)    The present invention provides food or preventive drugs whereby food residues retained in the digestive tract over a long period of time due to, for example, overeating even in a healthy person not suffering from constipation and having regular defecation can be discharged by elevating the transit speed without showing cathartic effects or unpleasant symptoms, thereby protecting the human body from the undesirable effects of putrefactive and toxic metabolites and enabling health enhancement and the prevention of colon cancer.

Namely, use of one or more members selected from among indigestible saccharides, low-viscosity and water-soluble dietary fibers and magnesium compounds in an adequate amount makes it possible to shorten maximum transit time through the digestive tract and prevent colon cancer without causing any cathartic effect, loose stools or unpleasant side effects. Moreover, transit time through the digestive tract can be shortened by using adequate amounts of lactulose and magnesium oxide without causing any cathartic effect, loose stools or unpleasant side effects.

**Description**

TECHNICAL FIELD

The present invention relates to techniques for preventing ingested food from being retained in the digestive tract, particularly in the lower digestive tract (large intestine), for a long period of time, and for shortening the time required from food ingestion to discharge from the human body without accompanying cathartic effects and loose stool, in particular, for shortening the time required to excrete feces with the slowest transit speed, i.e., the feces evacuated last among food residues taken in a single meal (coprostasis) from the human body, thereby inhibiting ill effects caused by harmful putrefactive metabolites produced from food residues in the digestive tract.

BACKGROUND ART

Burkitt et al. has indicated that Westerners, whose diets have a higher content of processed foods with little dietary fiber, have longer transit times for food residues through the digestive tract and have a higher incidence of large intestinal diseases such as colon cancer, etc. which are presumably attributed thereto, whereas Africans, who have unprocessed diets with abundant dietary fiber, have shorter transit times through the digestive tract and are almost free from large intestinal diseases. Further, Cummings et al. investigated groups in 23 districts throughout the world, and revealed that there is a negative correlation between the incident rate of colon cancer and fecal output. An investigation conducted by Burkitt et al. has already confirmed that there is a negative correlation between the fecal output and transit time through the digestive tract. Given this, the larger fecal output is, the shorter the transit time through the digestive tract becomes, thereby reducing incidence of colon cancer.

The longer the transit time of food residues through the lower digestive tract is, the more putrefaction of food residues by intestinal microflora proceeds, thereby producing more harmful putrefactive metabolites. For this reason, it is thought to be desirable that the transit time of food residue through the lower digestive tract be made as short as possible.

Conditions which lack regular bowel movements are usually diagnosed as constipation. More specifically, constipation is often diagnosed based on fecal frequency, such as the absence of regular bowel movements, and the numbers of days a week where bowel movements are absent, that is, the frequency of bowel movements. Constipation is not therefore diagnosed based on the subjective symptoms of the food residues transit time through the digestive tract. Coprostasis is defined as the feces being congested in the intestinal tract when the stomach and intestines are overburdened beyond gastrointestinal capacity (Mitsuo Kohda, Ph.D., "Choshoku o nuitara konatta (What happened if breakfast was not taken)", Shunjusha). Status and level of fecal congestion in the digestive tract is not thought to be presumed simply by the frequency of bowel movements as conventionally used for the diagnosis of constipation, but the status and level of fecal congestion need to be examined directly.

Even when constipation is not diagnosed, food residues may remain in the digestive tract for a long period of time, when food residues pass slowly through the digestive tract and are excreted little by little every day; however, a long transit time through the digestive tract, as mentioned above, is not desirable in view of the putrefactive metabolites produced from food residues. Given this, to assess the production level of harmful putrefactive metabolites from food residues retained for a long period of time in a healthy person who is not constipated, transit time through the digestive tract needs to be examined aside from the diagnosis of constipation.

Time required for food ingested to be excreted, i.e. transit time for food residues through the digestive tract, is predicted to be variable depending on factors such as diet, age, physical conditions, etc. even among persons who have regular bowel movements and are considered healthy by the diagnosis criteria for constipation. Various diseases are likely to be induced, as also indicated earlier by Burkitt in that a long transit time through the digestive tract increases the incidence rate of large intestinal diseases. For this reason, when food residues transit slowly through the digestive tract over a long period of time while a large amount of harmful putrefactive metabolites are produced, even a healthy person who defecates every day may be affected in the intestinal tract and the living body. However, since little is known about the relationship between digestive tract transit time and the production of harmful putrefactive metabolites, medically objective criteria with respect to a suitable time required from ingestion to excretion of food is also not known. Feces which travel the slowest through the digestive tract and are the last to be defecated after a single meal are thought to contain a larger amount of accumulated harmful putrefactive metabolites. Such feces can be well referred to as coprostasis, as defined by Mitsuo Kohda. The large intestinal membrane in contact with coprostasis is exposed to the highest concentration of harmful putrefactive metabolites for a long time, increasing the risk of colon cancer. The time required to excrete food residues with the slowest transit speed through the digestive tract is the maximum transit time through the digestive tract.

Laxatives and cathartic drugs may effectively be used to increase the frequency of bowel movements in treating constipation patients; however they are not necessarily said to be effective in shortening transit time or maximum transit time through the digestive tract of food residues that have been retained for a long period of time. Further, laxatives or cathartic drugs, used on a healthy person not suffering from constipation, often cause discomfort such as abdominal bloating, etc. and weakness from indigestion. Agents for ameliorating maximum transit time through the digestive tract and agents for ameliorating transit time through digestive tract, capable of maintaining and promoting the health of a healthy person and of preventing various diseases caused by food residues being retained in the digestive tract for a long period of time, by easily shortening transit time and maximum transit time through the digestive tract without the above symptoms have not been known.

Indigestible saccharides and dietary fibers are known to work well for constipation and the improvement of fecal status because they are not digested in the upper digestive tract, but are fermented and metabolized by intestinal microflora when such materials reach the lower digestive tract (large intestine), increases fecal volume, etc; however, these studies suggested the improvement of fecal status or constipation mainly based on increased fecal volume and shortened intervals between bowel movements, but did not suggest the improvement of transit time and maximum transit time through the digestive tract.

In particular, it is thoroughly examined that indigestive oligosaccharides cause proliferation of the *Bifidobacterium* in the large intestine, making the intestinal environment acidic by the action of the thus generated short-chain fatty acids, thereby improving intestinal microflora. It is understood that conditions with a decreased level of *Bifidobacterium* in the large intestine due to moderate constipation or some causes can be improved to attain decreased harmful intestinal putrefactive metabolites and improved fecal status by administrating oligosaccharides to increase *Bifidobacterium* levels, whereby lowered intestinal pH inhibits the proliferation of other harmful intestinal microflora for a better intestinal environment. In particular, lactitol and lactulose, indigestible saccharides, are known to reduce the ammonia produced by harmful intestinal microflora, and are hence used as pharmaceutical agents to treat symptoms accompanying intestine functional improvement and hyperammonemia. For hyperammonemia, 19.5 to 39 g of lactulose powder having a 97% or higher purity is given per adult per day, and 18 to 36 g of lactulose powder having a 100% purity is given in 3 portions a day per adult. Lactitol and lactulose are given in said high doses to hepatic encephalopathy patients and those whose intestinal functions are decreased and are hence susceptible to colonic polyps. However, it is not recommended to use the same dose of lactitol or lactulose with a healthy person whose intestinal functions are not diminished but has a longer retention time of food residues in the digestive tract simply affected by eating habits such as over-eating, because said drugs cause discomfortable symptoms such as a strong cathartic effect, weakness after defecation due to abdominal bloating and indigestion, etc. When 3 g of lactulose a day is taken, statistically significant increased number of the *Bifidobacterium,* lowered pH, increased moisture contents, reduced putrefactive metabolites, lowered β-glucuronidase activity, lowered nitroreductase activity, and lowered azoreductase activity are observed in feces; however, whether or not food residues, which have a long transit time through the digestive tract and produce large amounts of putrefactive metabolites while being retained over an extended period of time in the digestive tract, are easily evacuated has not been examined. Therefore, no study about the influence indigestive saccharides and dietary fibers cause on the transit time through the digestive tract of a healthy person not suffering from constipation is known.

Magnesium oxide sold as a cathartic drug is listed as a therapeutic drug for constipation in Japanese Pharmacopoeia 14th Edition, and described as "it should be taken as a laxative 2g a day, either in 3 portions before or after meals, or in a single portion before sleeping". 2g of magnesium oxide are equivalent to 1160 mg in terms of magnesium. When magnesium oxide in such an amount is used on a healthy person who is not constipated and has regular bowel movements with simply a long transit time or a maximum transit time through the digestive tract, its drastic cathartic action causes side effects such as weakness after defecation, etc. due to a sensation of abdominal bloating and indigestion, thereby making the use thereof undesirable. Japanese Pharmacopoeia does not show the usage or a dose of magnesium oxide for a healthy person, who is not constipated but has a long transit time or a maximum transit time through the digestive tract, to improve transit time through the digestive tract while maintaining normal defecation conditions free from loose stool, without a cathartic effect and other side effects.

The Unexamined Japanese Patent Publication No. 2001-48792 discloses a cathartic drug containing as an active ingredient activated magnesium oxide having a specific surface area (BET) of 21 $m^2$/g or more. In an example of said publication, a liquid preparation containing 2.4 g of activated magnesium oxide per 100 ml was given to 5 subjects in an amount of 70 ml (974 mg in terms of magnesium) a day, and its cathartic drug effect was shown. Further, 100 ml of an oligosaccharide solution and 40 ml of a prune-containing drinking water was added to 100 ml of a liquid preparation containing 2.4 g of activated magnesium oxide per 100 ml in order to prepare a liquid preparation, which was given to 5 subjects in an amount of 150 ml (870 mg in terms of magnesium) a day, whereby its cathartic effect was shown.

Usage and dose of pharmaceutical products are usually prescribed to treat a patient only during a disease, but cannot be prescribed to a healthy person for daily use for the purpose of preventing disease. There is no single study, including Unexamined Japanese Patent Publication No. 2001-48792, which thoroughly examined that magnesium oxide shortens food residues retention time in a healthy person not suffering from constipation, without an accompanying cathartic action or loose stools.

[Patent Document 1]

Unexamined Japanese Patent Publication No. 2001-48792

DISCLOSURE OF THE INVENTION

PROBLEMS TO BE SOLVED BY THE INVENTION

It is therefore desired that food residues retained in the digestive tract of a healthy person, not suffering from constipation, over an extended period of time be easily defecated without using a cathartic drug, which causes loose stools. In particular, it is demanded that food residues having the slowest transit speed be evacuated in a shorter time (improvement of maximum transit time through the digestive tract (improvement of coprostasis)).

An object of the present invention is to promote health maintenance of a healthy person not suffering from constipation by smoothly excreting food residues retained in the digestive tract over a long period of time due to overeating, etc. without causing cathartic effects or loose stools, thereby reducing the influence of harmful putrefactive metabolites produced in the intestines of the human body.

MEANS FOR SOLVING THE PROBLEMS

The present inventors found that when a healthy person not suffering from constipation and having regular bowel movements takes at least one member selected from indigestible saccharides, low-viscosity and water-soluble dietary fibers and magnesium compounds, particularly lactulose and magnesium oxide together, maximum transit time and transit time through the digestive tract extended by overeating, etc. can be shortened without unpleasant side effects such as bloating sensation, cathartic effect, loose stools, etc., whereby the present invention has been accomplished.

More specifically, the present invention relates to the following:

Article 1. An agent for ameliorating maximum transit time through the digestive tract comprising at least any one of indigestible saccharides, low-viscosity and water-soluble dietary fibers and magnesium compounds.

Article 2. An agent for ameliorating maximum transit time according to Article 1, wherein the indigestive saccharide is at least one member selected from the group consisting of lactulose, galacto-oligosaccharide, raffinose, stachyose, lactosucrose, fructo-oligosaccharide, isomalto-oligosaccharide, xylo-oligosaccharide, paratinose oligosaccharide, difructose anhydride III, sorbitol, maltitol, lactitol, reduced paratinose, and reduced starch syrups; the low-viscosity and water-soluble dietary fiber is at least one member selected from the group consisting of indigestive dextrin, partially hydrolyzed guar gum, polydextrose, water-soluble soybean polysaccharides, low molecular weight alginic acid, and water-soluble corn fiber; and the magnesium compound is at least one member selected from the group consisting of magnesium oxide, magnesium hydroxide, magnesium silicate, and magnesium carbonate.

Article 3. An agent for ameliorating maximum transit time according to Article 2 comprising lactulose and magnesium oxide.

Article 4. An agent for ameliorating maximum transit time according to any of Articles 1 to 3, wherein the agent further comprises a calcium compound.

Article 5. An agent for ameliorating transit time comprising lactulose and magnesium oxide.

Article 6. An agent for ameliorating transit time according to Article 5, wherein the agent further comprises a calcium compound.

Article 7. A preventive agent for colon cancer comprising lactulose and magnesium oxide.

Article 8. A preventive agent for colon cancer according to Article 7, wherein the agent further comprises a calcium compound.

Article 9. A method for shortening maximum transit time through the digestive tract comprising orally administering any one of indigestive saccharides, low-viscosity water-soluble dietary fibers and magnesium compounds in an amount effective in improving maximum transit time through the digestive tract.

Article 10. A method for shortening maximum transit time through the digestive tract according to Article 9, wherein the oral dosages per adult a day of indigestive saccharides, low-viscosity water-soluble dietary fibers and magnesium compounds are 0.8 to 6 g, 1 to 10 g and 40 to 600 mg on a magnesium basis, respectively.

Article 11. A method for shortening maximum transit time through the digestive tract according to Articles 9 or 10, wherein the indigestive saccharide is at least one member selected from the group consisting of lactulose, galacto-oligosaccharide, raffinose, stachyose, lactosucrose, fructo-oligosaccharide, isomalto-oligosaccharide, xylo-oligosaccharide, paratinose oligosaccharide, difructose anhydride III, sorbitol, maltitol, lactitol, reduced paratinose, and reduced starch syrups; the low-viscosity and water-soluble dietary fiber is at least one member selected from the group consisting of indigestive dextrin, partially hydrolyzed guar gum, polydextrose, water-soluble soybean polysaccharides, low molecular weight alginic acid, and water-soluble corn fiber; and the magnesium compound is at least one member selected from the group consisting of magnesium oxide, magnesium hydroxide, magnesium silicate, and magnesium carbonate.

Article 12. A method for preventing colon cancer comprising orally administering any one of indigestive saccharides, low-viscosity water-soluble dietary fibers, and magnesium compounds in an amount effective in preventing colon cancer.

Article 13. A method for preventing colon cancer according to Article 12, wherein oral dosages per adult a day of indigestive saccharides, low-viscosity water-soluble dietary fibers and magnesium compounds are 0.8 to 6 g, 1 to 10 g and 40 to 600 mg on a magnesium basis, respectively.

Article 14. A method for preventing colon cancer according to Article 12 or 13, wherein the indigestive saccharide is at least one member selected from the group consisting of lactulose, galacto-oligosaccharide, raffinose, stachyose, lactosucrose, fructo-oligosaccharide, isomalto-oligosaccharide, xylo-oligosaccharide, paratinose oligosaccharide, difructose anhydride III, sorbitol, maltitol, lactitol, reduced paratinose, and reduced starch syrups; the low-viscosity and water-soluble dietary fiber is at least one member selected from the group consisting of indigestive dextrin, partially hydrolyzed guar gum, polydextrose, water-soluble soybean polysaccharides, low molecular weight alginic acid, and water-soluble corn fiber; and the magnesium compound is at least one member selected from the group consisting of magnesium oxide, magnesium hydroxide, magnesium silicate, and magnesium carbonate.

Article 15. A method for shortening transit time through the digestive tract comprising orally administering lactulose and magnesium oxide in an amount effective in improving transit time through the digestive tract.

Article 16. A method for shortening transit time through the digestive tract according to Article 15 comprising oral dosages per adult a day of lactulose and magnesium oxide of 0.8 to 6 g, and 40 to 600 mg on a magnesium bases, respectively.

Article 17. Use of any one of indigestive saccharides, low-viscosity water-soluble dietary fibers, and magnesium oxides for shortening maximum transit time through the digestive tract.

Article 18. Use according to Article 17, wherein the indigestive saccharide is at least one member selected from the group consisting of lactulose, galacto-oligosaccharide, raffinose, stachyose, lactosucrose, fructo-oligosaccharide, isomalto-oligosaccharide, xylo-oligosaccharide, paratinose oligosaccharide, difructose anhydride III, sorbitol, maltitol, lactitol, reduced paratinose, and reduced starch syrups; the low-viscosity and water-soluble dietary fiber is at least one member selected from the group consisting of indigestive dextrin, partially hydrolyzed guar gum, polydextrose, water-soluble soybean polysaccharides, low molecular weight alginic acid, and water-soluble corn fiber; and the magnesium compound is at least one member selected from the group consisting of magnesium oxide, magnesium hydroxide, magnesium silicate, and magnesium carbonate.

Article 19. Use of any one of indigestive saccharaides, low-viscosity water-soluble dietary fibers and magnesium compounds for preventing colon cancer.

Article 20. Use according to Article 19, wherein the indigestive saccharide is at least one member selected from the group consisting of lactulose, galacto-oligosaccharide, raffinose, stachyose, lactosucrose, fructo-oligosaccharide, isomalto-oligosaccharide, xylo-oligosaccharide, paratinose oligosaccharide, difructose anhydride III, sorbitol, maltitol, lactitol, reduced paratinose, and reduced starch syrups; the low-viscosity and water-soluble dietary fiber is at least one member selected from the group consisting of indigestive dextrin, partially hydrolyzed guar gum, polydextrose, water-soluble soybean polysaccharides, low molecular weight alginic acid, and water-soluble corn fiber; and the magnesium compound is at least one member selected from the group consisting of magnesium oxide, magnesium hydroxide, magnesium silicate, and magnesium carbonate.

Article 21. Use of lactulose and magnesium oxide for shortening transit time through the digestive tract.

In the specification, the improvement of maximum transit time through the digestive tract means the shortening of maximum transit time through the digestive tract, and is synonymous with the improvement of coprostasis.

EFFECT OF THE INVENTION

According to the present invention, maximum transit time and transit time through the digestive tract can be shortened without unpleasant side effects such as a sensation of abdominal bloating, cathartic effects, loose stools, etc., particularly a bloating sensation, and adverse effects to the human body by harmful putrefactive metabolites produced from food residues are inhibited, whereby health is maintained or enhanced by the prevention of colon cancer, etc. More specifically, the present invention can provide about 17 to about 70 hours of transit time through the digestive tract, and about 25 to about 100 hours of maximum transit time through the digestive tract, without causing a sensation of abdominal bloating. Further, the present invention is capable of reducing variation range between each meal (standard deviation) of the maximum transit time and the transit time through the digestive tract, thereby preventing food residues from being retained in the digestive tract over an extremely long period of time.

BEST MODE FOR CARRYING OUT THE INVENTION

In the present invention, transit time, particularly maximum transit time, through the digestive tract can be shortened without causing a sensation of abdominal bloating by using any of indigestible saccharides, low-viscosity water-soluble dietary fibers and magnesium compounds, especially the combination of lactulose and magnesium oxide. Food residues from a single meal are usually not evacuated all at once, but excreted in several defecations. The term, maximum transit time through the digestive tract, used herein means the time required from food ingestion, in a single meal, to the excretion of food residues that have the slowest transit speed. Further, the term, transit time through the digestive tract, means the average time from food ingestion to defecation after a single meal. Furthermore, the amelioration of maximum transit time through the digestive tract and the amelioration of transit time through the digestive tract mean the shortening of maximum transit time through the digestive tract and the shortening of transit time through the digestive tract, respectively. In the present specification, the improvement of coprostasis includes the shortening of maximum transit time through the digestive tract.

The extent of the shortening of the maximum transit time through the digestive tract is not limited, but times from about 40 to about 150 hours before administering the amelioration agent of the present invention are preferably shortened to about 25 to about 100 hours after administration. The extent of the shortening of the transit time through the digestive tract is not limited, but times from about 30 to about 120 hours before administering the amelioration agent of the present invention are preferably shortened to about 17 to about 70 hours after administration.

The indigestible saccharides used in the present invention are not limited, and examples include oligosaccharides such as lactulose, galacto-oligosaccharide, raffinose, lactosucrose, fructo-oligosaccharide, isomalto-oligosaccharide, xylo-oligosaccharide, stachyose, paratinose oligosaccharide, difructose anhydride III, etc.; and sugar alcohols such as sorbitol, maltitol, lactitol, reduced paratinose, reduced starchy syrup, etc. Also usable are prune, extract thereof and like natural materials abundant with indigestible saccharides and extracts thereof. Preferable among these are lactulose, galacto-oligosaccharide, fructo-oligosaccharide, isomalto-oligosaccharide, sorbitol, maltitol, lactitol, and reduced starchy syrup. These indigestible sacchirides can be used singly, or two or more can be used in combination. The intake of these indigestible saccharides per healthy person not suffering from constipation per day is preferably 0.8 to 6 g, and more preferably 1.0 to 5.0 g. When intake is within this range, maximum transit time through the digestive tract and transit time through the digestive tract are shortened, and variation range between each meal can be further reduced. Furthermore, cathartic effects, abdominal bloating sensation, gas production, indigestion, and like side effects are drastically suppressed.

The low-viscosity water-soluble dietary fibers used in the present invention are the kind of dietary fibers that have a low-viscosity in a state of being dissolved in water among water-soluble dietary fibers, and are not limited so long as the viscosity of a 1 % aqueous solution thereof is 100 mPa · sec. or lower when measured at a solution temperature of 20 °C using a type B viscometer. Examples of low-viscosity water-soluble dietary fibers include indigestible dextrin, partially hydrolyzed guar gums, polydextrose, water-soluble soybean polysaccharides, low molecular weight alginic acid, water-soluble corn fiber, etc. Preferable among these are indigestible dextrin and partially hydrolyzed guar gums. These low-viscosity water-soluble dietary fibers can be used singly, or two or more can be used in combination. The intake thereof per healthy person not suffering from constipation is preferably 1 to 10 g, and more preferably 2.0 to 8.0 g a day.

The magnesium compounds used in the present invention include magnesium oxide, magnesium hydroxide, magnesium silicate, magnesium carbonate, magnesium chloride, magnesium sulfate, trimagnesium phosphate, dolomite, etc., and magnesium oxide, magnesium hydroxide, magnesium silicate, magnesium carbonate are preferable because they are not deliquescent and are free of the bitterness distinctive in magnesium. In the present invention, they can be used singly, or two or more can be used in combination. The intake thereof per healthy person not suffering from constipation is preferably 40 to 600 mg, and more preferably 80 to 500 mg, on a magnesium basis, a day. When an intake is within this range, transit time through the digestive tract and maximum transit time through the digestive tract are shortened, and variation range between each meal thereof can be further reduced. Furthermore, cathartic effects, abdominal bloating sensation, gas production, indigestion, and like side effects are drastically suppressed.

The indigestible saccharides, low-viscosity water-soluble dietary fibers and magnesium compounds used in the agent for ameliorating maximum transit time through the digestive tract, the agent for ameliorating transit time through the digestive tract and the preventive agent for colon cancer of the present invention are preferably contained in a proportion of 0.05 to 80 % by weight of the indigestible saccharides in the entire amount of the composition, 0.05 to 80 % by weight of the low-viscosity water-soluble dietary fibers in the entire amount of the composition, and 0.004 to 50 % by weight, on a magnesium basis, of the magnesium compound in the entire amount of the composition.

The agent for ameliorating maximum transit time through the digestive tract, the agent for ameliorating transit time through the digestive tract and the preventive agent for colon cancer of the present invention are preferably administered once a day, early in the morning on an empty stomach or before going to bed; however the daily dose may be administered in two or more portions. The intake according to the present invention is based on an average healthy adult not suffering from constipation, and can be adjusted as necessary depending on usage, age, sex, degrees of other diseases, life style, and other conditions.

According to the present invention, calcium compounds can further be contained in the agent for ameliorating the maximum transit time through the digestive tract, the agent for ameliorating transit time through the digestive tract and the preventive agent for colon cancer. Calcium compounds have an effect in alleviating the tendency to loose stools caused by magnesium oxide, and is hence desirably contained in the agents and preventive agent. The calcium compounds to be used in the present invention include calcium carbonate, calcium chloride, calcium phosphate, calcium lactate, calcium gluconate, calcined shell calcium, egg shell calcium, dolomite, etc. The intake of a calcium compound a day is preferably 10 to 1200 mg in terms of the amount of calcium. The proportion of a calcium compound contained in the agent for ameliorating maximum transit time through the digestive tract, the agent for ameliorating transit time through the digestive tract and the preventive agent for colon cancer of the present invention is, in terms of the amount of calcium, preferably 0.004 to 20 % by weight, and more preferably 0.03 to 10 % by weight.

The agent for ameliorating maximum transit time through the digestive tract, the agent for ameliorating transit time through the digestive tract and the preventive agent for colon cancer of the present invention comprises at least any one of indigestible saccharides, low-viscosity water-soluble dietary fibers and magnesium compounds; however two or more of them can be used in combination if required. In particular, when a magnesium compound is used in combination with indigestible saccharides and/or water-soluble dietary fibers, the amelioration of maximum transit time through the digestive tract is enhanced, and is hence desirable. Particularly, when lactulose and magnesium oxide are used in combination, maximum transit time through the digestive tract is shortened, especially the maximum transit time through the digestive tract when taking a high calorie diet can be shortened along with reduction of variation range between each meal, and side effects are drastically suppressed. For this reason, the combination of lactulose and magnesium oxide is the most preferable.

When lactulose and magnesium oxide are used in combination, the intake of lactulose per healthy adult not suffering from constipation a day is preferably 0.8 to 6.0 g, and more preferably 1.0 to 5.0 g. Lactulose contained in the agent for ameliorating maximum transit time through the digestive tract, the agent for ameliorating transit time through the digestive tract and the preventive agent for colon cancer of the present invention is preferably in a proportion of 0.05 to 80 % by weight, and more preferably in a proportion of 0.2 to 60 % by weight. The intake of magnesium oxide per healthy adult not suffering from constipation a day is, in terms of the amount of magnesium, preferably 40 to 600 mg, and more preferably 80 to 500 mg. The magnesium oxide contained in the agent for ameliorating maximum transit time through the digestive tract, the agent for ameliorating transit time through the digestive tract and the preventive agent for colon cancer of the present invention is preferably in a proportion of 0.004 to 50 % by weight, and is more preferably in a proportion of 0.01 to 40 % by weight. Further, the agent for ameliorating maximum transit time through the digestive tract, the agent for ameliorating transit time through the digestive tract and the preventive agent for colon cancer containing a combination of lactulose and magnesium oxide can further contain other indigestive saccharides, low-viscosity water-soluble dietary fibers, and magnesium compounds.

The agent for ameliorating maximum transit time through the digestive tract, the agent for ameliorating transit time through the digestive tract and the preventive agent for colon cancer of the present invention include orally administrable compositions. Examples include forms such as pharmaceutical agents, food, supplements, etc.

The form of dosage unit for the agent for ameliorating maximum transit time through the digestive tract, the agent for ameliorating transit time through the digestive tract and the preventive agent for colon cancer is not limited insofar as it is suitable for oral administration, and can be suitably selected depending on the purpose of administration. Specific examples include oral agents such as tablets, coated tablets, powders, granules, capsules, solutions, pills, suspensions, emulsions, etc. These agents are prepared by standard production processes known in this field.

When the agent for ameliorating maximum transit time through the digestive tract, the agent for ameliorating transit time through the digestive tract and the preventive agent for colon cancer are used in the form of food, indigestible saccharides, low-viscosity water-soluble dietary fibers and/or magnesium compounds are combined with edible carriers, food ingredients, food additives, etc., in accordance with the form as necessary for the preparation by a standard process. Examples of such food forms include liquid foods such as beverages, etc.; solid foods such as tablets, granules, chewable tablets, etc.; or the like. Alternatively, they can be used in the form of semisolid food such as yogurt, etc. They can further be used as health foods, functional foods, designated health foods, nutrition functional foods, foods for sick people, etc. Specific examples of such food forms include liquid beverages such as juices, soft drinks, teas, etc.; powdered beverages such as powdered juices, powdered soups, etc.; snacks such as chocolates, candies, chewing gums, ice creams, jellies, cookies, biscuits, corn flakes, chewable tablets, film sheets, wafers, gummies, rice crackers, buns with bean-paste filling, etc.; seasonings such as dressings, sauces, etc.; breads, pastas, konjakmannans, fish pastes (kamaboko, etc.), seasoned sprinkles, oral sprays, troches, etc. Live microorganism such as lactic acid bacteria, inactivated microorganisms, other probiotics, vitamins, botanical medicines, plants themselves such as herbs, extracts thereof, etc., may further be added as additives.

Examples of the carrier comprised in food include sugar alcohols such as xylitol and erythritol, etc.; excipients such as crystalline cellulose, lactose, white sugar, glucose, starch, carbonates, phosphates, etc.; binders such as gelatin, alginic acid, xanthane gum, cellulose, hydroxypropylcellulose, methylcellulose, carrageenan, pullulan, pectin, etc.; emulsifiers such as sucrose esters of fatty acids, sorbitan esters of fatty acids, enzymatically treated lecithin, enzymatically decomposed lecithin, saponin, etc.; antioxidants such as ascorbic acid, tocopherol, etc.; acidifiers such as lactic acid, citric acid, gluconic acid, glutamic acid, etc.; fortifiers such as vitamins, amino acids, lactate, citrate, gluconate, etc.; anti-caking agents such as silicon dioxide, etc.; lubricants such as sucrose esters of fatty acids, stearates, etc.; sweeteners such as sucralose, acesulfame potassium, aspartame, glycyrrhizin, etc.; and flavorings. Thickening polysaccharides and dietary fibers can be contained to an extent so far as they do not affect the amelioration effects on transit time through the digestive tract.

When the agent for ameliorating maximum transit time through the digestive tract, the agent for ameliorating transit time through the digestive tract and the preventive agent for colon cancer are used in the form of pharmaceutical products, indigestible saccharides, low-viscosity water-soluble dietary fibers and/or magnesium compounds are combined with pharmaceutically acceptable carriers, pharmaceutical materials, additives, etc., in accordance with the form as necessary for preparation by a standard process. Examples of pharmaceutical forms include solutions, solid preparations such as tablets, granules, parvules, powders, and orally administrable forms such as capsules or the like in which said solutions or solid preparations are encapsulated, etc.

Examples of pharmaceutically acceptable carriers include excipients such as lactose, white sugar, sodium chloride, glucose, starch, calcium carbonate, kaolin, crystalline cellulose, silicic acid, methylcellulose, glycerin, sodium alginate, gum arabic, talc, calcium hydrogen phosphate, dibasic calcium phosphate, dibasic sodium phosphate, dipotassium hydrogen phosphate, potassium dihydrogen phosphate, calcium dihydrogen phosphate, sodium dihydrogen phosphate, calcium sulfate, calcium lactate, cacao oil, etc.; binders such as simple syrup, glucose solution, starch solution, gelatin solution, polyvinyl alcohol, polyvinyl ether, polyvinyl pyrrolidone, cross polyvinyl pyrrolidone, hydroxypropylcellulose, low-substituted hydroxypropylcellulose, hydroxypropylmethylcellulose, hydroxyethylcellulose, carboxy vinyl polymer, crystalline cellulose, powdered cellulose, crystalline cellulose carmellose sodium, carboxymethylcellulose, shellac, methylcellulose, ethyl cellulose, potassium phosphate, powdered gum arabic, pullulan, dextrin, maize starch, alpha-starch, hydroxypropyl starch, gelatin, xanthane gum, tragacanth, powdered tragacanth, macro goal, etc.; disintegrants such as dry starch, sodium alginate, agar powder, laminaran powder, sodium bicarbonate, calcium carbonate, polyoxyethylene sorbitan esters of fatty acids, sodium lauryl sulfate, stearic acid monoglyceride, starch, lactose, etc.; disintegration inhibitors such as white sugar, stearic acid, cacao butter, hydrogenated oil, etc.; moisturizers such as glycerin, starch, etc.; adsorbents such as starch, lactose, kaolin, bentonite, colloidal silicic acid, etc.; lubricants such as purified talc, stearate, boric acid powder, polyethylene glycol, etc. Further, tablets can be coated with typical coatings as necessary to prepare, e.g. sugar-coated tablets, gelatin film-coated tablets, enteric-coated tablets, film-coated tablets, double layer tablets, multi-layer tablets, etc. Capsules are prepared by mixing active ingredients and various carriers exemplified above, and filled into hard gelatin capsules, soft gelatin capsules, or the like.

Liquid preparations may be in the form of water-based or oil-based suspensions, solutions, syrups, or elixirs, and can be prepared by standard methods, using typical carriers, additives, etc. Thickening polysaccharides and dietary fibers can further be added to such an extent that they do not affect the amelioration effect on transit time through the digestive tract.

Examples of flavoring include peppermint oil, eucalyptus oil, cinnamon oil, fennel oil, clove oil, orange oil, lemon oil, rose oil, fruit flavor, mint flavor, peppermint powder, dl-menthol, and l-menthol, etc.

Subjects for administration of the agent for ameliorating maximum transit time through the digestive tract, the agent for ameliorating transit time through the digestive tract and the preventive agent for colon cancer of the present invention include those who are healthy and are concerned about bowel conditions. The term, those who are concerned about bowel conditions means healthy people who are not diagnosed as having constipation according to the diagnosis criteria for constipation and have regular bowel movements, but whose stool is harder and fecal weight is smaller each day than those with normal bowel habits. Therefore, the present invention does not include subjects for administration who are constipated. Those who are concerned about bowel conditions used herein have more often than 0.5 but under 1.0 bowel movement a day, those who are healthy with normal bowel habits have 1.0 bowel movement or more a day, and those who are constipated lack regular bowel movements and have bowel movements under 0.43 a day.

Further, according to our tests, those on a high-calorie diet have longer maximum transit time through the digestive tract than those on a low-calorie diet, and have wide variation of maximum transit time through the digestive tract for each meal (i.e., greater variation range between each meal). The combination of lactulose and magnesium oxide is useful for shortening maximum transit time through the digestive tract and reducing the variation ranges between each meal, particularly, for those on a high-calorie diet. Consequently, preferable subjects for administration of the agent for ameliorating maximum transit time through the digestive tract, the agent for ameliorating transit time through the digestive tract and the preventive agent for colon cancer containing lactulose and magnesium oxide of the present invention, are those on a high-calorie diet. A high-calorie diet used herein means calorie intake per adult of 35 kcal or more per kg body weight, or about 2100 kcal or more, per day.

The present invention is described in detail in reference to EXAMPLES below, but is not limited to these EXAMPLES.

EXAMPLE

Test Example 1

The method proposed by Hinton et al. was invented to measure the time required from food ingestion to defecation, namely, transit time through the digestive tract. More specifically, a plurality of radio-opaque markers were taken with a meal, and the time required from food ingestion to defecation (ti) and the number of markers contained in the evacuated feces (xi) were inspected based on x-ray images of the collected feces, followed by calculation of transit time through the digestive tract using the following formula. Transit time through the digestive tract indicates an average time required for a plurality of radio-opaque markers taken simultaneously to be evacuated.

$$\text{Transit time through the digestive tract} = \frac{\sum_{i=1}^{i=n} x_i\, t_i}{\sum_{i=1}^{i=n} x_i}$$

A test sample in a dose shown in Table 1, together with 15 markers, were administered per day with meals to a healthy subject (1 person) not suffering from constipation, and the markers in the feces of the subject were identified thereafter. Test samples were administered for 3 days, and during which the transit times through the digestive tract of 5 or 6 meals were measured. Transit time through the digestive tract calculated by the above formula after each meal and the time required for the last marker to be evacuated (maximum transit time through the digestive tract) were recorded. Table 1 shows the average transit time through the digestive tract, obtained by totaling each transit time through the digestive tract after each meal and dividing the sum by the number of meals; and the average maximum transit time through the digestive tract, obtained by totaling each maximum transit time through the digestive tract after each meal and dividing the sum by the number of meals. Statistical treatment was performed using t-tests. In addition to the marker identification, the fecal weight and defecation frequency were recorded, and daily average fecal weight and defecation frequency were calculated, and shown in Table 1. In the column of transit time through the digestive tract, the upper lines show average transit time through the digestive tract, the lower lines show average maximum transit time through the digestive tract, and the values in the parenthesis on the immediate right indicate standard deviations thereof.

Table 1

| Test Sample | Dose/ Day | Transit Time Maximum Transit Time through Digestive Tract (unit: hour) Standard Deviation in parentheses | t-Test ($P$ value) **$P <0.01$ *$P <0.05$ | Average Fecal Weight (g/day) | Average Defecation Frequency (times/day) |
|---|---|---|---|---|---|
| No Intake (Control) | Not Administered | 33.3 (9.1) 51.2 (16.8) | - | 177 | 1.4 |
| Locust Bean Gum | 5 g | 26.4 (7.6) 37.6 (13.3) | (0.087) (0.088) | 324 | 1.5 |
| Psyllium | 6 g | 27.8 (5.6) 50.3 (12.4) | (0.105) (0.455) | 274 | 1.5 |
| Beet Fiber | 4 g | 27.7 (3.9) 45.4 (10.2) | (0.108) (0.259) | 189 | 2.0 |

(continued)

| Test Sample | Dose/ Day | Transit Time Maximum Transit Time through Digestive Tract (unit: hour) Standard Deviation in parentheses | t-Test (*P* value) **P <0.01 *P <0.05 | Average Fecal Weight (g/day) | Average Defecation Frequency (times/day) |
|---|---|---|---|---|---|
| Lactulose | 1 g | 21.8 (3.1) 34.4 (9.4) | ** * | 315 | 1.8 |
| Raffinose | 2 g | 22.2 (2.5) 31.0 (11.5) | * * | 243 | 2.3 |
| Lactosucrose | 1.5 g | 19.7 (3.5) 31.3 (8.6) | ** * | 267 | 2.0 |
| Fructo-oligosaccharide | 2.8 g | 20.7 (3.5) 28.8 (7.6) | ** ** | 314 | 2.0 |
| Isomalto-Oligosaccharide | 1.2 g | 18.5 (2.5) 28.8 (7.5) | ** ** | 278 | 2.0 |
| Xylo-oligosaccharide | 0.9 g | 17.2 (3.4) 28.2 (8.3) | ** ** | 220 | 2.0 |
| Soybean Oligosaccharide | 1.2 g | 22.3 (2.4) 31.4 (8.6) | * * | 268 | 2.3 |
| Lactosucrose Isomalto-oligosaccharide | 0.4 g 0.6 g | 22.9 (3.1) 31.1 (8.5) | ** ** | 254 | 2.0 |
| Galacto-oligosaccharide | 1 g | 20.5 (2.0) 38.5 (14.4) | ** (0.087) | 246 | 2.0 |
| Indigestible Dextrin | 4.5 g | 20.2 (2.6) 33.3 (8.9) | ** * | 269 | 2.0 |
| Magnesium Oxide | 0.17 g | 20.6 (4.1) 38.1 (10.1) | ** * | 274 | 2.3 |
| Magnesium Oxide | 0.35 g | 17.5 (3.1) 32.4 (7.6) | ** * | 204 | 2.0 |
| Magnesium Oxide | 0.50 g | 21.6 (6.8) 35.6 (6.9) | * * | 313 | 2.3 |
| Magnesium Oxide | 0.75 g | 21.0 (4.3) 34.9 (7.2) | ** * | 235 235 | 2.3 2.3 |
| Magnesium Oxide | 1.0g | 22.9 (2.8) 37.1 (8.2) | * (0.054) | 414 | 2.0 |
| Oxide Magnesium Oxide | 1.5g | 19.5 (1.9) 35.6 (6.4) | ** * | 426 | 2.3 |
| Magnesium Sulfate | 4.5g | 20.2 (2.4) 35.8 (10.1) | ** * | 389 | 2.3 |

(continued)

| Test Sample | Dose/ Day | Transit Time Maximum Transit Time through Digestive Tract (unit: hour) Standard Deviation in parentheses | t-Test (*P* value) **\*\*P <0.01 \*P <0.05** | Average Fecal Weight (g/day) | Average Defecation Frequency (times/day) |
|---|---|---|---|---|---|
| Magnesium Oxide | 1.0g | 21.5 (5.3) | ** | 286 | 2.3 |
| Calcium Carbonate | 1.0g | 29.7 (8.6) | ** | | |
| Magnesium Oxide | 1.0g | 25.6 (7.2) | (0.053) | 272 | 1.7 |
| Calcium Carbonate | 3.0g | 36.9 (10.4) | * | | |
| Galacto-oligosaccharide | 0.83g | 21.7 (5.2) | ** | 249 | 2.5 |
| Magnesium Oxide | 0.08g | 35.8 (9.1) | * | | |
| Lactulose | 1g | 19.5 (3.6) | ** | 275 | 2.3 |
| Magnesium Oxide | 0.5g | 31.7 (9.0) | * | | |
| Partially hydrolyzed guar gum | 4.0g | 22.5 (5.1) 30.8 (7.8) | * ** | 270 | 2.3 |
| Maltitol | 1.6g | 22.0 (3.5) | * | 246 | 1.7 |
| Reduced Starch Syrup | 0.5g | 34.6 (9.1) | * | | |

When 0.9 to 2.8 g a day of indigestible saccharides were administered, shortened transit time and maximum transit time through the digestive tract and shortened standard deviations thereof were observed, in comparison with no administration (control). When 4.5 g or 4.0 g a day of indigestible dextrin or partially hydrolyzed guar gum, i.e., low-viscosity water-soluble dietary fibers, were administered respectively, shortened transit time and maximum transit time through the digestive tract and shortened standard deviations thereof were observed, in comparison with no administration (control). During the period of ingesting these test samples, the subjects experienced no significant increase in average fecal amount, i.e., no diarrhea or loose stools were observed.

On the contrary, when dietary fibers, such as locust bean gum, psyllium and beet fiber, which are thought to be efficacious in improving fecal status, were administered, statistically significant shortening of transit time and maximum transit time through the digestive tract were not observed. Locust bean gum and psyllium are water-soluble dietary fibers, but have characteristics of increasing viscosity when dissolved in water. Beet fiber is an insoluble dietary fiber. Increased average fecal weights were observed when locust bean gum and psyllium were used; however they had no shortening effect on the transit time and maximum transit time through the digestive tract. Increased average defecation frequency was observed when beet fiber was used; however it had no shortening effect on transit time and maximum transit time through the digestive tract. Some case studies have conventionally presumed that the transit time of entire food residues through the digestive tract would be shortened based on the improvement effects on fecal status, i.e. increased fecal weight and defecation frequency (bowel movements per unit time); however, the above results indicate that transit time and maximum transit time through the digestive tract cannot be presumed based simply on the improvement effects on fecal status when the typical diet is supplemented with indigestible saccharides and dietary fibers.

When 0.17 to 1.5 g of magnesium oxide a day was administered, shortened transit time and maximum transit time through the digestive tract were observed, in comparison with no administration (control). The average fecal weight was not increased extremely and loose stools were not caused at up to 0.75 g of magnesium oxide intake a day. 1.0 to 1.5 g intake of magnesium oxide a day greatly increased, i.e. more than doubled, the average fecal weight, and caused loose stools having high a moisture content. However, when 1.0 g of magnesium oxide was taken together with 1.0 g or 3.0 g of calcium carbonate, the average fecal weight did not increase much and loose stools were not caused. Given this, the upper limit of magnesium oxide intake a day is desirably 1.0 g. Further, the addition of magnesium oxide to lactulose was advantageous in that such unpleasant symptoms as abdominal bloating sensation, gas production, etc. were unexpectedly more alleviated and feces were moderately softer for easy defecation than when lactulose was taken singly. Considering all of the above, the effective amount of magnesium oxide a day is 0.08 to 1.0 g, and 46 to 580 mg on a magnesium basis, since magnesium oxide materials typically contain 58 % of magnesium. Improvement effects on the transit time through the digestive tract are rendered by magnesium in magnesium sulfate; however, magnesium sulfate has stronger cathartic effects than magnesium oxide, thereby causing a tendency towards loose stools.

Test Example 2

The following ingredients were homogenously mixed without pre-processing in a V-type mixer to prepare a soft drink powder, of which 3.2 g was dissolved and dispersed in 200 ml of water before being taken. Thirteen healthy women who were not diagnosed as being constipated and including those who were concerned about bowel habits took the soft drink powder once a day for two weeks, and changes in the transit time and maximum transit time through the digestive tract before and during ingestion were inspected in the same manner as in Test Example 1. Ten radio-opaque markers in three different configurations were serially taken with breakfast, lunch and supper, and all feces were collected from the following day to inspect transit time, maximum transit time through the digestive tract, and fecal weights based on the radiography of the feces. The three meals served to the test participants on the day of radio-opaque markers administration were all the same commercial meal menus. Since the magnesium oxide commercially distributed as a food additive typically contains 58 % of magnesium, 1 g of lactulose and 300 mg of magnesium were taken in a single intake of 3. 2 g of the soft drink powder. Table 2 shows average transit time and average maximum transit time through the digestive tract of three meals before and during ingestion of the soft drink powder.

| Soft Drink Powder Materials | |
| --- | --- |
| Ingredients | Composition (% by weight) |
| Lactulose (97 % purity) | 36.0 |
| Mangesium Oxide | 16.2 |
| Citric Acid Anhydride | 25.5 |
| Sodium Acetate Anhydride | 9.0 |
| Calcium Gluconate | 6.5 |
| Sucralose | 0.5 |
| Powdered Flavoring | 4.5 |
| Xanthane gum . | 1.8 |
| Total | 100.0 |

Table 2

| Subject No. | Transit Time through Digestive Tract (unit: hour) | | Maximum Transit Time through Digestive Tract (unit: hour) | |
| --- | --- | --- | --- | --- |
| | Before Ingestion | During Ingestion | Before Ingestion | During Ingestion |
| 1 | 124.1 | 55.7 | 151.3 | 97.5 |
| 2 | 58.3 | 46.9 | 73.8 | 66.5 |
| 3 | 77.2 | 45.1 | 88.3 | 57.8 |
| 4 | 44.8 | 23.0 | 58.4 | 42.9 |
| 5 | 103.1 | 45.9 | 136.3 | 59.7 |
| 6 | 43.4 | 52.4 | 60.1 | 63.6 |
| 7 | 38.7 | 32.8 | 52.3 | 41.5 |
| 8 | 36.6 | 32.6 | 58.5 | 45.7 |

(continued)

| Subject No. | Transit Time through Digestive Tract (unit: hour) | | Maximum Transit Time through Digestive Tract (unit: hour) | |
|---|---|---|---|---|
| | Before Ingestion | During Ingestion | Before Ingestion | During Ingestion |
| 9 | 90.9 | 69.6 | 116.0 | 86.7 |
| 10 | 70.1 | 43.6 | 70.1 | 57.6 |
| 11 | 92.3 | 40.5 | 109.9 | 82.2 |
| 12 | 43.9 | 32.3 | 58.5 | 55.7 |
| 13 | 59.4 | 49.7 | 74.6 | 73.3 |
| Average | 67.9 | 43.9 | 85.2 | 63.9 |
| Standard Deviation | 27.9 | 12.1 | 32.8 | 17.1 |
| t-test P value | 0.003 | | 0.005 | |

As shown in Table 2, except for one subject, specifically Subject No.6, the transit times through the digestive tract were shortened during the ingestion period of the soft drink. Thirteen subjects had a transit time through the digestive tract of about 37 to about 124 hours before taking the soft drink; however, the transit time was reduced to about 23 to about 70 hours during the ingestion of the soft drink. When the average of 13 subjects were compared, a transit time of 67.9 hours through the digestive tract before taking the soft drink was reduced to 43.9 hours during the period of taking the same. The transit time though the digestive tract was improved at a statistically significant level of below 1 %.

Similarly, the maximum transit times through the digestive tract of 12 subjects out of 13, excluding for one, Subject No. 6, were shortened during the period of taking the soft drink. Thirteen subjects had a maximum transit time through the digestive tract of about 52 to about 151 hours, but these were reduced to about 42 to about 98 during the period of taking the soft drink. When the averages of 13 subjects were compared, 85.2 hours for the maximum transit time through the digestive tract before taking the soft drink was reduced to 63.9 hours during the period of taking the same at a statistically significant level of below 1 %.

The average fecal weight a day of 13 subjects was increased to 117.3 g during the period of taking the soft drink from 76.2 g before taking the soft drinks at a statistically significant level of below 5 %. The shortening effects on transit time and maximum transit time through the digestive tract were more remarkable among subjects having had longer transit times and maximum transit times before taking the soft drink. Side effects and unpleasant symptoms such as diarrhea, soft stools, the sensation of abdominal bloating were not observed during the period of taking the soft drink. Therefore, the soft drink powder can shorten transit times of food residues retained in the digestive tract over a long period without pain and discomfort caused by cathartic action.

Out of 13 subjects, fecal samples of subjects Nos. 1 to 7 were able be stored at a low temperature immediately after defecation, and total putrefactive metabolite concentration, ammonia concentration, pH, and moisture content were further measured. The total putrefactive metabolites were analyzed using a gas chromatograph to assay phenol, p-cresol, 4E-phenol, indole and skatole, thereby taking the total value thereof as the total putrefactive metabolite concentration. Table 3 shows the results of quantitative measurement of the total putrefactive metabolite concentrations and ammonia concentrations. Table 4 shows fecal pH and moisture content.

Table 3

| Subject No. | Total Putrefactive Metabolite Concentration (Unit: $\mu$g/g) | | Ammonia Concentration (Unit:$\mu$g/g) | |
|---|---|---|---|---|
| | Before Ingestion | During Ingestion | Before Ingestion | During Ingestion |
| 1 | 123.3 | 68.0 | 1250 | 672 |
| 2 | 74.2 | 26.0 | 1041 | 445 |
| 3 | 165.0 | 58.9 | 671 | 577 |
| 4 | 59.6 | 57.5 | 611 | 615 |
| 5 | 168.6 | 31.6 | 1218 | 509 |
| 6 | 115.1 | 81.0 | 1013 | 702 |

(continued)

| Subject No. | Total Putrefactive Metabolite Concentration (Unit: $\mu$g/g) | | Ammonia Concentration (Unit:$\mu$g/g) | |
|---|---|---|---|---|
| | Before Ingestion | During Ingestion | Before Ingestion | During Ingestion |
| 7 | 118.1 | 58.2 | 1000 | 633 |
| Average | 117.7 | 54.5 | 972 | 593 |
| Standard Deviation | 41.1 | 19.4 | 246.9 | 90.8 |
| t-test P Value | 0.0099 | | 0.0095 | |

Table 4

| Subject No. | pH | | Moisture Content (%) | |
|---|---|---|---|---|
| | Before Ingestion | During Ingestion | Before Ingestion | During Ingestion |
| 1 | 6.8 | 7.2 | 72.4 | 77.0 |
| 2 | 5.8 | 6.8 | 74.3 | 81.6 |
| 3 | 6.6 | 7.1 | 63.7 | 78.3 |
| 4 | 6.6 | 7.5 | 68.2 | 78.6 |
| 5 | 7.3 | 7.2 | 67.4 | 83.9 |
| 6 | 6.3 | 7.0 | 75.4 | 78.6 |
| 7 | 6.4 | 6.9 | 76.6 | 83.8 |
| Average | 6.5 | 7.1 | 71.1 | 80.2 |
| Standard Deviation | 0.4 | 0.2 | 4.8 | 2.8 |
| t-test P Value | 0.0055 | | 0.0029 | |

As shown in Table 3, all subjects had a reduced total putrefactive metabolite concentration while taking the soft drink in comparison with before taking the same. The average total putrefactive metabolite concentration of 7 subjects was reduced from 117.7 $\mu$g/g before taking the soft drink to 54.4 $\mu$g/g while taking the same at a statistically significant level of below 1 %. Further, the average ammonia concentration of 7 subjects was reduced from 972 $\mu$g/g before taking the soft drink to 593 $\mu$g/g while taking the same at a statistically significant level of below 1 %. These results demonstrate that a preventive effect for colon cancer can be expected according to the present invention.

Contrarily, as shown in Table 4, moisture content was increased during ingestion of the soft drink, compared to before taking the same. The average of 7 subjects was increased from 71.1 % before taking the soft drink to 80.2 % while taking the same at a statistically significant level of below 1 %. The moisture content of each subject during ingestion of the soft drink was not such that unpleasant symptoms such as loose stools or watery stools were caused.

Further, since fecal pH was elevated from about 6.5 before ingestion to 7.1 during ingestion, intestinal pH was confirmed to have been elevated. On the other hand, in the study of ameliorating constipation using dietary fibers or oligosaccharides, these components are known to be beneficial because intestinal pH is lowered due to the conversion of dietary fibers and oligosaccharides to short-chain fatty acids by intestinal microflora, because of which the intestinal environment is acidified, thereby suppressing the overgrowth of detrimental intestinal microflora and production of putrefactive metabolites. Given this, it is suggested that the shortening effects on transit time and maximum transit time through the digestive tract by orally administered lactulose and magnesium oxide are different from the constipation-ameliorating effects rendered by dietary fibers and oligosaccharides. Further, changes in the total putrefactive metabolite concentration before and during ingestion were calculated based on weights of dried feces after moisture was removed. The total putrefactive metabolite concentrations were reduced, and it was revealed that the weight reductions were not simply caused by the dilution effects of increased fecal moisture content.

According to the results above, the inhibition effects on fecal putrefactive metabolites of, in particular, lactulose and magnesium oxide are thought to be primarily due to the shortening effects of transit time and maximum transit time through the digestive tract, but not due to the acidification of the intestinal environment or the dilution effects of an increased fecal moisture level which have been conventionally thought to attribute to such effects.

Test Example 3

The same soft drink powder as prepared in Test Sample 2 was taken once a day for two weeks by 29 healthy female subjects who were free of constipation, and the occurrence of uncomfortable symptoms during ingestion was observed. Table 5 shows the results. No subject experienced an abdominal bloating sensation accompanying pain. Nine subjects reported some intestinal gas production (faintly noticed: 8 subjects, noticed: 1 subject), but the production was so slight that it was not troublesome. One subject reported an occurrence of light diarrhea, but the symptoms were negligible and not near the level of a problem.

Table 5

| Uncomfortable Symptom | Numbers of Subjects Reporting Uncomfortable Symptoms Out of 29 Subjects | |
|---|---|---|
| | Faintly Noticed (numbers of subject) | Noticed (numbers of subject) |
| Abdominal Bloating Sensation with Pain | 0 | 0 |
| Excreting Gas | 8 | 1 |
| Diarrhea | 1 | 0 |

Consequently, according to the present invention, average transit time and maximum transit time through the digestive tract can be improved without accompanying discomfort, such as an abdominal bloating sensation, etc.

Test Example 4

A solution having the composition shown in Table 6 below was prepared. After preparation, the solution was pasteurized at 95 °C for 2 minutes. 100 ml thereof was filled into a bottle that could be hermetically sealed at 85 °C or higher, the bottle was cap-sealed, and then cooled with running water. A bottle of the solution provides 2 g of indigestible saccharide and 336 mg of magnesium per 100 ml.

Table 6

| | |
|---|---|
| Lactulose Syrup (50% purity) | 4.0g |
| Concentrated Clear Apple Juice (5 times concentration) | 2.35g |
| Citrate (anhydrous) | 2.2g |
| Gluconic Acid Solution 50% | 1.3g |
| DL-Malic Acid | 0.7g |
| Magnesium Oxide | 0.58g |
| Calcium Gluconate | 0.33g |
| L-Ascorbic Acid | 0.20g |
| Flavoring | 0.13g |
| Sucralose | 0.01g |
| Refined water | 91.90g |
| Total (volume conversion) | 103.70g (100 ml) |

100 ml of the above solution a day was administered to a subject (1 healthy male), and high-calorie meals were provided for 3 days (6 meals) . The transit time and maximum transit time through the digestive tract were determined in the same manner as in Test Example 1. Cases with high-calorie meals and low-calorie meals without administrating the solution were similarly tested. The same subject participated in all cases with, e.g., high-calorie meals, low-calorie meals, and the solution administration. Further, fecal weights and defecation frequencies were recorded to calculate an average fecal weight and an average defecation frequency per day. Table 7 shows the results. The high-calorie meals consisted of 4 bags of retort curry (221 kcal/bag), 5 packs of boiled rice (302 kcal/pack), and 80 g of cereal (150 kcal), totaling 2694 kcal a day. The low-calorie meal consisted of 2 bags of retort curry, 3 packs of boiled rice, and 40 g of cereal, totaling 1498 kcal a day. The weight of the high-calorie meal was 1920 g, and that of the low-calorie meal was 1060 g.

Table 7

| Meal Ingested | Upper : Transit Time through Digestive Tract Lower : Maximum Transit Time through Digestive Tract | | | Average Fecal Weight (g/day) | Average Defecation Frequency (time/day) |
|---|---|---|---|---|---|
| | Average (Hours) | t-Test P Value | Standard Deviation | | |
| High-Calorie Meal | 24.0 43.6 | - - | 5.5 9.7 | 226.3 | 1.3 |
| High-calorie Meal + Solution | 17.0 30.1 | 0.014 0.018 | 3.3 8.5 | 351.3 | 2.0 |
| Low-Calorie Meal | 24.3 39.2 | 0.465 0.193 | 2.9 4.7 | 146.0 | 2.7 |

The transit times through the digestive tract showed little difference between the high-calorie meal and the low-calorie meal cases. The maximum transit time through the digestive tract was shorter with the low-calorie meal than the high-calorie meal. When the subject was on the high-calorie diet, the standard deviation of the transit time through the digestive tract and the maximum transit time through the digestive tract was greater than that of when the subject was on the low-calorie diet. Namely, high-calorie intake causes a greater variation range between each meal of the transit time through the digestive tract and maximum transit time through the digestive tract, indicating higher possibilities of extremely long fecal retention. Magnesium and lactulose are capable of shortening the transit time through the digestive tract and maximum transit time through the digestive tract in the case of overeating, especially shortening the retention time of feces, which are evacuated last, are the most putrefactive in the intestine, and hence presumed to have the highest risk of causing colon cancer.

EXAMPLE 1

A jelly having the following composition was prepared by a standard method. The ingredients were dissolved in refined water by heating, and pasturized at 95 °C for 2 minutes. 100 ml of the treated jelly was filled in a plastic cup to which an aluminum seal was applied, and then cooled with running water. The jelly contained 250 mg of magnesium per 100 ml.

| Ingredients | Composition (% by weight) |
|---|---|
| Magnesium Carbonate | 1.1 |
| Citric Acid Crystal | 1.5 |
| 50 % Gluconic Acid | 1.0 |
| High Fructose Corn Syrup | 12.0 |
| Cloudy Grapefruit Juice Concentrate | 10.0 |
| Gelling Agents (Agar and Carragheenan) | 1.0 |
| Flavoring | 0.1 |
| Refined water | 73.3 |
| Total | 100.0 |

EXAMPLE 2

A ground green tea having the following composition was prepared. The ingredients were prepared in the form of granules using a fluidized-bed granulator. 5 g of the powdered tea, when dissolved in 180 ml of hot water, provided 2 g of indigestible dextrin and 1.5 g of difructose anhydride III.

| Ingredients | Composition (% by weight) |
|---|---|
| Indigestible Dexitrin (90 % Purity) | 44.5 |
| Difructose Anhydride III (97 % Purity) | 31.0 |

(continued)

| Ingredients | Composition (% by weight) |
|---|---|
| Ground Green Tea | 24.5 |
| Total | 100.0 |

EXAMPLE 3

Tablets having the following composition were prepared. After preparation, the tablets were pressed using a rotary tablet press so as to have a weight of 320 mg per tablet. 4 to 10 tablets a day provided about 0.85 to 2.1 g of indigestible saccharide and about 186 to 464 mg of magnesium.

| Ingredients | Composition (% by weight) |
|---|---|
| Fructo-oligosaccharide (95 % Purity) | 50.5 |
| Magnesium Oxide | 25.5 |
| Maltitol Powder (93.5 % or higher Purity) | 20.0 |
| Sucrose Esters of Fatty Acids | 5.0 |
| Total | 100.0 |

EXAMPLE 4

A soft drink having the following composition was prepared by a standard method. After preparation, the soft drink was pasteurized at 95 °C for 2 minutes. 160 g of the treated drink was filled into a tin can at 90 °C or higher, the can was cap-sealed, and then cooled with running water. A can of the soft drink (160 g) was able to provide 5.3 g of indigestible saccharide.

| Ingredients | Composition (% by weight) |
|---|---|
| Isomalto-oligosaccharide Syrup (50 % content) | 3.0 |
| Sorbitol (90 % Purity) | 2.0 |
| Citric Acid Crystals | 0.5 |
| Sodium Citrate | 0.05 |
| High Fructose Corn Syrup | 10.0 |
| Flavoring | 0.15 |
| Refined Water | 84.3 |
| Total | 100.0 |

EXAMPLE 5

A jelly having the following composition was prepared by a standard method. 100 g of the jelly contained 255 mg of magnesium and 50 mg of calcium.

| Ingredients | Composition (% by weight) |
|---|---|
| Magnesium Oxide | 0.44 |
| Citric Acid Crystals | 1.0 |
| 50 % Gluconic Acid | 0.5 |
| Calcium Gluconate (98 % content) | 0.5 |
| High Fructose Corn Syrup | 2.0 |
| Cloudy Grapefruit Juice Concentrate | 5.0 |
| Flavoring | 0.15 |
| Agar | 0.50 |
| Carragheenan | 0.40 |
| Refined Water | 79.51 |
| Total | 100.0 |

INDUSTRIAL APPLICABILITY

The present invention can be advantageously utilized in fields which require the shortening of the maximum transit time through the digestive tract and transit time through the digestive tract. The present invention can also be used in fields which require the prevention of colon cancer.

## Claims

1. An agent for ameliorating maximum transit time through the digestive tract comprising at least any one of indigestible saccharides, low-viscosity and water-soluble dietary fibers and magnesium compounds.

2. An agent for ameliorating maximum transit time according to Claim 1, wherein the indigestive saccharide is at least one member selected from the group consisting of lactulose, galacto-oligosaccharide, raffinose, stachyose, lactosucrose, fructo-oligosaccharide, isomalto-oligosaccharide, xylo-oligosaccharide, paratinose oligosaccharide, difructose anhydride III, sorbitol, maltitol, lactitol, reduced paratinose, and reduced starch syrups; the low-viscosity and water-soluble dietary fiber is at least one member selected from the group consisting of indigestive dextrin, partially hydrolyzed guar gum, polydextrose, water-soluble soybean polysaccharides, low molecular weight alginic acid, and water-soluble corn fiber; and the magnesium compound is at least one member selected from the group consisting of magnesium oxide, magnesium hydroxide, magnesium silicate, and magnesium carbonate.

3. An agent for ameliorating maximum transit time according to Claim 2 comprising lactulose and magnesium oxide.

4. An agent for ameliorating maximum transit time according to any of Claims 1 to 3, wherein the agent further comprises a calcium compound.

5. An agent for ameliorating transit time comprising lactulose and magnesium oxide.

6. An agent for ameliorating transit time according to Claim 5, wherein the agent further comprises a calcium compound.

7. A preventive agent for colon cancer comprising lactulose and magnesium oxide.

8. A preventive agent for colon cancer according to Article 7, wherein the agent further comprises a calcium compound.

9. A method for shortening maximum transit time through the digestive tract comprising orally administering any one of indigestive saccharides, low-viscosity water-soluble dietary fibers and magnesium compounds in an amount effective in improving maximum transit time through the digestive tract.

10. A method for shortening maximum transit time through the digestive tract according to Claim 9, wherein the oral dosages per adult a day of indigestive saccharides, low-viscosity water-soluble dietary fibers and magnesium compounds are 0.8 to 6 g, 1 to 10 g and 40 to 600 mg on a magnesium basis, respectively.

11. A method for shortening maximum transit time through the digestive tract according to Claim 9 or 10, wherein the indigestive saccharide is at least one member selected from the group consisting of lactulose, galacto-oligosaccharide, raffinose, stachyose, lactosucrose, fructo-oligosaccharide, isomalto-oligosaccharide, xylo-oligosaccharide, paratinose oligosaccharide, difructose anhydride III, sorbitol, maltitol, lactitol, reduced paratinose, and reduced starch syrups; the low-viscosity and water-soluble dietary fiber is at least one member selected from the group consisting of indigestive dextrin, partially hydrolyzed guar gum, polydextrose, water-soluble soybean polysaccharides, low molecular weight alginic acid, and water-soluble corn fiber; and the magnesium compound is at least one member selected from the group consisting of magnesium oxide, magnesium hydroxide, magnesium silicate, and magnesium carbonate.

12. A method for preventing colon cancer comprising orally administering any one of indigestive saccharides, low-viscosity water-soluble dietary fibers, and magnesium compounds in an amount effective in preventing colon cancer.

13. A method for preventing colon cancer according to Claim 12, wherein oral dosages per adult a day of indigestive saccharides, low-viscosity water-soluble dietary fibers and magnesium compounds is 0.8 to 6 g, 1 to 10 g and 40 to 600 mg on a magnesium basis, respectively.

**14.** A method for preventing colon cancer according to Claim 12 or 13, wherein the indigestive saccharide is at least one member selected from the group consisting of lactulose, galacto-oligosaccharide, raffinose, stachyose, lactosucrose, fructo-oligosaccharide, isomalto-oligosaccharide, xylo-oligosaccharide, paratinose oligosaccharide, difructose anhydride III, sorbitol, maltitol, lactitol, reduced paratinose, and reduced starch syrups; the low-viscosity and water-soluble dietary fiber is at least one member selected from the group consisting of indigestive dextrin, partially hydrolyzed guar gum, polydextrose, water-soluble soybean polysaccharides, low molecular weight alginic acid, and water-soluble corn fiber; and the magnesium compound is at least one member selected from the group consisting of magnesium oxide, magnesium hydroxide, magnesium silicate, and magnesium carbonate.

**15.** A method for shortening transit time through the digestive tract comprising orally administering lactulose and magnesium oxide in an amount effective in improving transit time through the digestive tract.

**16.** A method for shortening transit time through the digestive tract according to Claim 15 comprising oral dosages per adult a day of lactulose and magnesium oxide of 0.8 to 6 g, and 40 to 600 mg on a magnesium bases, respectively.

**17.** Use of any one of indigestive saccharides, low-viscosity water-soluble dietary fibers, and magnesium oxides for shortening maximum transit time through the digestive tract.

**18.** Use according to Claim 17, wherein the indigestive saccharide is at least one member selected from the group consisting of lactulose, galacto-oligosaccharide, raffinose, stachyose, lactosucrose, fructo-oligosaccharide, isomalto-oligosaccharide, xylo-oligosaccharide, paratinose oligosaccharide, difructose anhydride III, sorbitol, maltitol, lactitol, reduced paratinose, and reduced starch syrups; the low-viscosity and water-soluble dietary fiber is at least one member selected from the group consisting of indigestive dextrin, partially hydrolyzed guar gum, polydextrose, water-soluble soybean polysaccharides, low molecular weight alginic acid, and water-soluble corn fiber; and the magnesium compound is at least one member selected from the group consisting of magnesium oxide, magnesium hydroxide, magnesium silicate, and magnesium carbonate.

**19.** Use of any one of indigestive saccharaides, low-viscosity water-soluble dietary fibers and magnesium compounds for preventing colon cancer.

**20.** Use according to Claim 19, wherein the indigestive saccharide is at least one member selected from the group consisting of lactulose, galacto-oligosaccharide, raffinose, stachyose, lactosucrose, fructo-oligosaccharide, isomalto-oligosaccharide, xylo-oligosaccharide, paratinose oligosaccharide, difructose anhydride III, sorbitol, maltitol, lactitol, reduced paratinose, and reduced starch syrups; the low-viscosity and water-soluble dietary fiber is at least one member selected from the group consisting of indigestive dextrin, partially hydrolyzed guar gum, polydextrose, water-soluble soybean polysaccharides, low molecular weight alginic acid, and water-soluble corn fiber; and the magnesium compound is at least one member selected from the group consisting of magnesium oxide, magnesium hydroxide, magnesium silicate, and magnesium carbonate.

**21.** Use of lactulose and magnesium oxide for shortening transit time through the digestive tract.

## INTERNATIONAL SEARCH REPORT

| | International application No. |
|---|---|
| | PCT/JP2005/021272 |

A. CLASSIFICATION OF SUBJECT MATTER
*A61K31/7016*(2006.01), *A61K31/702*(2006.01), *A61K31/7032*(2006.01),
*A61K31/715*(2006.01), *A61K31/047*(2006.01), *A61K33/06*(2006.01), *A61P1/10*
(2006.01), *A61P35/00*(2006.01), *A23L1/30*(2006.01), *A23L1/304*(2006.01)
According to International Patent Classification (IPC) or to both national classification and IPC

B. FIELDS SEARCHED

Minimum documentation searched (classification system followed by classification symbols)
*A61K31/047*(2006.01), *A61K31/7016*(2006.01)-*A61K31/7032*(2006.01),
*A61K31/715*(2006.01)-*A61K31/739*(2006.01), *A61K33/06*(2006.01)-*A61K33/12*
(2006.01), *A23L1/30*(2006.01)-*A23L1/305*(2006.01)

Documentation searched other than minimum documentation to the extent that such documents are included in the fields searched
Jitsuyo Shinan Koho          1922-1996    Jitsuyo Shinan Toroku Koho    1996-2005
Kokai Jitsuyo Shinan Koho    1971-2005    Toroku Jitsuyo Shinan Koho    1994-2005

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 62-220169 A  (Yakult Honsha Co., Ltd.),<br>28 September, 1987 (28.09.87),<br>& EP 307523 A1          & US 4859488 A<br>& KR 9202004 B1 | 1,2<br>4 |
| X<br>Y | JP 63-63366 A  (Iwatani & Co., Ltd.),<br>19 March, 1988 (19.03.88),<br>(Family: none) | 1,2<br>4 |
| X<br>Y | JP 1-319421 A  (Asahi Chemical Industry Co.,<br>Ltd.),<br>25 December, 1989 (25.12.89),<br>(Family: none) | 1,2<br>4 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>02 February, 2006 (02.02.06) | Date of mailing of the international search report<br>28 February, 2006 (28.02.06) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2005/021272

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 5-255097 A (Kabushiki Kaisha Miyarisan Seibutsu Igaku Kenkyusho), 05 October, 1993 (05.10.93), (Family: none) | 1,2<br>4 |
| X<br>Y | JP 5-255092 A (Kyowa Hakko Kogyo Co., Ltd.), 05 October, 1993 (05.10.93), (Family: none) | 1,2<br>4 |
| X<br>Y | JP 2000-50843 A (Morinaga Milk Industry Co., Ltd.), 22 February, 2000 (22.02.00), (Family: none) | 1,2,4<br>3,5,6 |
| X<br>Y | JP 2001-97868 A (Nippon Tensai Seito Kabushiki Kaisha), 10 April, 2001 (10.04.01), (Family: none) | 1,2<br>3-6 |
| X<br>Y | JP 2002-51731 A (Kabushiki Kaisha Toyo Shin'yaku), 19 February, 2002 (19.02.02), (Family: none) | 1,2<br>3-6 |
| X<br>Y | JP 2002-104980 A (Kabushiki Kaisha Takama), 10 April, 2002 (10.04.02), (Family: none) | 1,2<br>4 |
| X<br>Y | JP 2002-223726 A (Ribatepu Seiyaku Kabushiki Kaisha), 13 August, 2002 (13.08.02), (Family: none) | 1,2<br>4 |
| X<br>Y | JP 2004-137194 A (Fancl Corp.), 13 May, 2004 (13.05.04), & WO 03/090759 A1 & EP 1504761 A1 & KR 2004106367 A | 1,2<br>4 |
| X<br>Y | JP 2004-166639 A (Kabushiki Kaisha Tsuboshi Seicha Honpo), 17 June, 2004 (17.06.04), (Family: none) | 1,2<br>3-6 |
| X<br>Y | JP 2004-224777 A (Kabushiki Kaisha Hayashibara Seibutsu Kagaku Kenkyujo), 12 August, 2004 (12.08.04), (Family: none) | 1,2<br>3-6 |
| X<br>Y | JP 2004-292356 A (Rohto Pharmaceutical Co., Ltd.), 21 October, 2004 (21.10.04), (Family: none) | 1,2<br>3-6 |

Form PCT/ISA/210 (continuation of second sheet) (April 2005)

## INTERNATIONAL SEARCH REPORT

International application No.

PCT/JP2005/021272

**Box No. II    Observations where certain claims were found unsearchable (Continuation of item 2 of first sheet)**

This international search report has not been established in respect of certain claims under Article 17(2)(a) for the following reasons:

1. ☒  Claims Nos.: 9-21
   because they relate to subject matter not required to be searched by this Authority, namely:
   Claims 9 to 21 involve methods for treatment of the human body or animal body by therapy under the provisions of the PCT Rule 39.1(iv).

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
   (See extra sheet.)

1. ☐  As all required additional search fees were timely paid by the applicant, this international search report covers all searchable claims.

2. ☐  As all searchable claims could be searched without effort justifying an additional fee, this Authority did not invite payment of any additional fee.

3. ☐  As only some of the required additional search fees were timely paid by the applicant, this international search report covers only those claims for which fees were paid, specifically claims Nos.:

4. ☒  No required additional search fees were timely paid by the applicant.   Consequently, this international search report is restricted to the invention first mentioned in the claims; it is covered by claims Nos.:
   A part of claim 1, a part of claim 2 and claims 3 to 6.

**Remark on Protest**
the

☐  The additional search fees were accompanied by the applicant's protest and, where applicable, payment of a protest fee..

☐  The additional search fees were accompanied by the applicant's protest but the applicable protest fee was not paid within the time limit specified in the invitation.

☐  No protest accompanied the payment of additional search fees.

Form PCT/ISA/210 (continuation of first sheet (2)) (April 2005)

| **INTERNATIONAL SEARCH REPORT** | International application No. |
| --- | --- |
| | PCT/JP2005/021272 |

Continuation of Box No.III of continuation of first sheet(2)

In claim 1, the active ingredient is described in the alternative form "one or more members selected from among indigestible saccharides, low-viscous and water-soluble dietary fibers and magnesium compounds". Since there is no matter common to all of these alternatives, the matter common to the group of the inventions as claimed in claim 1 resides in an agent for ameliorating the maximum passage time through the digestive tract (an agent for ameliorating retained fecal mass) containing some substance. However, it is a publicly known matter that some substance is usable as an agent for ameliorating the maximum passage time through the digestive tract (an agent for ameliorating retained fecal mass) without a need for citing any document and, therefore, this point cannot be considered as a technical feature making a contribution over prior art. Thus, it cannot be considered as a special technical feature as defined in Patent Cooperation Treaty Rule 13.2. Therefore, claim 1 does not comply with the requirement of unity of invention in international application. That is to say, claim 1 has three invention groups including: (1) an agent for ameliorating the maximum passage time through the digestive tract containing, as the active ingredient, "indigestible saccharides"; (2) an agent for ameliorating the maximum passage time through the digestive tract containing, as the active ingredient, "low-viscous and water-soluble dietary fibers"; and (3) an agent for ameliorating the maximum passage time through the digestive tract containing, as the active ingredient, "magnesium compounds".

Next, the unity of the inventions of the three invention groups as claimed in claim 1 and the inventions according to claims 3 to 8 will be discussed.

The matter common to all of these inventions resides in a composition containing some substance and acting on the digestive tract. Needless to cite any document, this point is also a publicly known matter and, therefore, cannot be considered a technical feature making a contribution over prior art. Thus, it cannot be considered as a special technical feature as defined in Patent Cooperation Treaty Rule 13.2. Therefore, the inventions according to claims 1 to 8 of the present international application do not comply with the requirement of unity of invention in international application.

(Scope of search concerning claim 1)

In claim 1, the active ingredient is expressed as "indigestible saccharides". Namely, it relates to an agent for ameliorating the maximum passage time through the digestive tract which contains saccharides defined by this property as the active ingredient. Although claim 1 involves any saccharides having this property, only small part of the claimed saccharides are disclosed in the meaning within PCT Article 5. Therefore, it appears that claim 1 is not supported by the disclosure of the description.

Even though the common technical knowledge at the point of the application is taken into consideration with respect to the expression "indigestible saccharides", the scope of the saccharides having the above property cannot be specified. Thus, claim 1 does not comply with the requirement of clearness in the meaning within PCT Article 6.

Concerning claim 1, therefore, the search was made on those containing the indigestible saccharides specified in claim 2 as the active ingredient.

Form PCT/ISA/210 (extra sheet) (April 2005)

## REFERENCES CITED IN THE DESCRIPTION

### Patent documents cited in the description

- JP 2001048792 A